(19) Europäisches Patentamt
European Patent Office
Office européen des brevets

(11) EP 4 684 729 A1

# (12) EUROPEAN PATENT APPLICATION

(43) Date of publication:
**28.01.2026 Bulletin 2026/05**

(21) Application number: **25191014.7**

(22) Date of filing: **22.07.2025**

(51) International Patent Classification (IPC):
***A61B 5/145*** *(2006.01)* ***A61B 5/1468*** *(2006.01)*
*A61B 5/053* *(2021.01)*

(52) Cooperative Patent Classification (CPC):
**A61B 5/1468; A61B 5/14532;** A61B 5/053;
A61B 5/14503; A61B 2562/12

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **22.07.2024 US 202463674023 P**
**20.06.2025 US 202519244390**

(71) Applicant: **Medtronic MiniMed, Inc.**
**Northridge, CA 91325-1219 (US)**

(72) Inventors:
- **KOSSAKOVSKI, Dmitri A.**
  **Northridge, 91325 (US)**
- **RAO, Ashwin K.**
  **Northridge, 91325 (US)**
- **GARAI, Ellis**
  **Northridge, 91325 (US)**
- **CHIU, Chia-Hung**
  **Northridge, 91325 (US)**
- **TRAN, Chi A.**
  **Mounds View, 55112 (US)**
- **ORTIZ, Andrew J.**
  **Somerville, 02145 (US)**

(74) Representative: **Zimmermann & Partner Patentanwälte mbB**
**P.O. Box 330 920**
**80069 München (DE)**

## (54) SYSTEMS AND METHODS FOR IMPROVING RUN-IN TIME, INTERFERENT REJECTION, AND LONGEVITY OF AN ANALYTE SENSOR

(57) An analyte sensor configured for fast run-in and interferent rejection includes a first working electrode including an analyte sensing molecule disposed thereon and configured to generate a first signal when exposed to an analyte; a second working electrode including an analyte sensing molecule disposed thereon and configured to generate a second signal when exposed to an analyte; a processor; and a memory. The memory, includes instructions which, when executed by the processor, cause the sensor to: measure the first sensor signal at the first working electrode; measure the second sensor signal at the second working electrode; determine a hand-off period for the sensor; and generate a fused sensor signal based on the first sensor signal for a first period of time and after the hand-off period, based on the second sensor signal for a second period of time.

EP 4 684 729 A1

## Description

CROSS-REFERENCE TO RELATED APPLICATIONS

This application claims the benefit of the filing date of provisional U.S. Patent Application No. 63/674,023, filed on July 22, 2024.

FIELD

The present disclosure relates generally to continuous glucose monitoring (CGM) and, more particularly, to analyte sensors and methods for improving run-in time, interferent rejection, and longevity of analyte sensors.

BACKGROUND

Analyte sensors such as biosensors include devices that use biological elements to convert a chemical analyte in a matrix into a detectable signal. There are many types of biosensors used for a wide variety of analytes, including amperometric glucose sensors for glucose level control for diabetes.

A typical glucose sensor works according to the following chemical reactions:

$$\text{Glucose} + O_2 \xrightarrow{\text{Glucose Oxidase}} \text{Gluconic acid} + H_2O_2, \quad \text{Equation 1;}$$

and

$$H_2O_2 \rightarrow O_2 + H_2^+ + 2e^-, \quad \text{Equation 2.}$$

The glucose oxidase is used to catalyze the reaction between glucose and oxygen to yield gluconic acid and hydrogen peroxide, $H_2O_2$, (Equation 1). The hydrogen peroxide reacts electrochemically as shown in Equation 2, and the current can be measured by a potentiostat. These reactions, which occur in a variety of oxidoreductases known in the art, are used in a number of sensor designs.

One common problem with analyte sensors is that they can electrochemically react not only with the analyte to be measured (or by-product of the enzymatic reaction with the analyte), but can also react with other electroactive chemical species that are not intentionally being measured, which causes an increase in signal strength due to these "interfering species" or "interferents." Typically, such interfering species are compounds with an oxidation or reduction potential that overlaps with the analyte to be measured (or a by-product of the enzymatic reaction with the analyte). For example, in a conventional amperometric glucose oxidase-based glucose sensor where the conventional sensor measures hydrogen peroxide, interfering species such as acetaminophen, ascorbate, and urate are known to confound true analyte signals, resulting in loss of sensor sensitivity and/or longevity.

Another common problem with analyte sensors is that they are less sensitive when insulin is injected near the analyte sensor. In view of the aforementioned shortcomings of analyte sensors, the present disclosure relates to an improvement in the design of analyte sensors to improve longevity and sensitivity in the proximity of an insulin bolus.

SUMMARY

In accordance with aspects of the present disclosure, an analyte sensor includes a first working electrode including an analyte sensing molecule disposed on the first working electrode and configured to generate a first signal when exposed to an analyte, and a second working electrode including an analyte sensing molecule disposed on the second working electrode and configured to generate a second signal when exposed to an analyte. The analyte sensor also includes a processor and a memory. The memory includes instructions which, when executed by the processor, cause the analyte sensor to measure the first sensor signal at the first working electrode and measure the second sensor signal at the second working electrode. The instructions, when executed by the processor, also cause the analyte sensor to determine a hand-off period for the analyte sensor and to generate a fused sensor signal based on using the first sensor signal for a first period of time and, after the hand-off period, based on the second sensor signal for a second period of time.

In an aspect of the present disclosure, the first working electrode may have a first platinum roughness, and the second working electrode may have a second platinum roughness that is different from the first platinum roughness.

In another aspect of the present disclosure, the first working electrode and the second working electrode may have at least one layer of a stack that differs between them.

In yet another aspect of the present disclosure, the first working electrode and the second working electrode may be operated independently, and wherein first working electrode and the second working electrode have a different operating potential than each other.

In a further aspect of the present disclosure, a geometric area of each working electrode may be scaled such that a sensor signal generated on each work electrode is equal when exposed to a given analyte value.

In yet a further aspect of the present disclosure, the hand-off period may be a predetermined period of time.

In an aspect of the present disclosure, the instructions, when executed by the processor, may further cause the analyte sensor to determine a first sensor glucose value based on the first sensor signal and determine a second sensor glucose value based on the second sensor signal. Determining the hand-off period may be based on a rate of change of the first sensor glucose value of the first working electrode being below a predetermined threshold.

In another aspect of the present disclosure, the working electrode with a higher surface area roughness may be located more distally on the analyte sensor relative to the other working electrode.

In a further aspect of the present disclosure, the instructions, when executed by the processor, may further cause the analyte sensor to determine at least one of an electrochemical impedance spectroscopy (EIS) parameter value or a conductivity value based on a fused sensor signal.

In accordance with aspects of the disclosure, the analyte sensor may include an interference rejection membrane on at least one of the first working electrode or the second working electrode.

In accordance with aspects of the disclosure, a processor-implemented method for operating an analyte sensor is presented. The method includes measuring a first sensor signal at a first working electrode including an analyte sensing molecule disposed on the first working electrode and configured to generate a first signal when exposed to an analyte, and measuring a second sensor signal at a second working electrode including an analyte sensing molecule disposed on the second working electrode and configured to generate a second signal when exposed to an analyte. The method also includes determining a hand-off period for the analyte sensor and generating a fused sensor signal based on using the first sensor signal for a first period of time and, after the hand-off period, based on the second sensor signal for a second period of time.

In a further aspect of the present disclosure, the method may further include determining a first sensor glucose value based on the first sensor signal. Determining the hand-off period may be based on a rate of change of the first sensor glucose value of the first working electrode being below a predetermined threshold.

In a further aspect of the present disclosure, the first working electrode and the second working electrode may have at least one layer of a stack that differs between them.

In yet a further aspect of the present disclosure, the method may further include independently operating the first working electrode and the second working electrode and the first working electrode and the second working electrode have a different operating potential than each other

In an aspect of the present disclosure, the second working electrode may have a different electrocatalytic activity than the first working electrode.

In an aspect of the present disclosure, the hand-off period may be a predetermined period of time.

In a further aspect of the present disclosure, the first working electrode may have a first platinum roughness, and the second working electrode may have a second platinum roughness that is different from the first platinum roughness.

In accordance with aspects of the disclosure, the method may further include determining at least one of an electrochemical impedance spectroscopy (EIS) parameter value or a conductivity value based on the fused sensor signal, and determining a presence of an interferent based on at least one of the EIS parameter value or the conductivity value.

In accordance with aspects of the disclosure, one or more non-transitory processor-readable media is presented. The non-transitory processor-readable media stores instructions which, when executed by one or more processors, cause performance of: measuring a first sensor signal at a first working electrode including an analyte sensing molecule disposed on the first working electrode and configured to generate a first signal when exposed to an analyte; and measuring a second sensor signal at a second working electrode including an analyte sensing molecule disposed on the second working electrode and configured to generate a second signal when exposed to an analyte. The instructions, when executed by the one or more processors, further cause performance of: determining a hand-off period for the analyte sensor; and generating a sensor signal based on the first sensor signal for a first period of time and, after the hand-off period, based on the second sensor signal for a second period of time.

Further disclosed herein is an analyte sensor configured for fast run-in and interferent rejection which includes a first working electrode including an analyte sensing molecule disposed thereon and configured to generate a first signal when exposed to an analyte; a second working electrode including an analyte sensing molecule disposed thereon and configured to generate a second signal when exposed to an analyte; a processor; and a memory. The memory, includes instructions which, when executed by the processor, cause the sensor to: measure the first sensor signal at the first working electrode; measure the second sensor signal at the second working electrode; determine a hand-off period for the sensor; and generate a fused sensor signal based on the first sensor signal for a first period of time and after the hand-off period, based on the second sensor signal for a second period of time.

The details of one or more aspects of the disclosure are set forth in the accompanying drawings and the description below. Other features, objects, and advantages of the techniques described in this disclosure will be apparent from the description and drawings, and from the claims.

## BRIEF DESCRIPTION OF THE DRAWINGS

A detailed description of aspects of the disclosure will be made with reference to the accompanying drawings, wherein like numerals designate corresponding parts in the figures.

FIG. 1 illustrates a perspective view of a subcutaneous sensor insertion set and a block diagram of an analyte sensor electronics device, in accordance with one or more aspects of this disclosure;
FIG. 2 illustrates a block diagram of an electronic circuit for sensing an output of an analyte sensor, in accordance with one or more aspects of this disclosure;
FIG. 3 illustrates a block diagram of an analyte sensor electronics device and a sensor including a plurality of electrodes, in accordance with one or more aspects of this disclosure;
FIG. 4 illustrates a sensor and a sensor electronics device, in accordance with an alternative aspect of this disclosure;
FIG. 5 is a graph illustrating two sensor signals from two respective working electrodes that have different electrocatalytic activity, in accordance with one or more aspects of this disclosure;
FIG. 6 is a graph illustrating a fused sensor signal based on the sensor signals of FIG. 6, in accordance with one or more aspects of this disclosure;
FIG. 7 is a diagram of a sensor of the device of FIG. 1 having two working electrodes that have different electrocatalytic activity, in accordance with one or more aspects of this disclosure;
FIG. 8 is a diagram illustrating a sensor of the device of FIG. 1 having an interference rejection membrane, in accordance with one or more aspects of this disclosure;
FIG. 9 is a diagram illustrating a sensor of the device of FIG. 1 having two different interference rejection membranes, in accordance with one or more aspects of this disclosure; and
FIG. 10 is a flow diagram illustrating a method for compensating for analyte interference of an analyte sensor, in accordance with aspects of this disclosure.

## DETAILED DESCRIPTION

In the following description, reference is made to the accompanying drawings which form a part hereof and which illustrate several aspects of the present disclosure. It is understood that other aspects may be utilized, and structural and operational changes may be made without departing from the scope of the present disclosure.

The aspects herein are described below with reference to flowchart illustrations of methods, systems, devices, apparatus, processor-executable products, processor-executable instructions, and programming and computer program products. It will be understood that each block of the flowchart illustrations, and combinations of blocks in the flowchart illustrations, can be implemented by programming instructions, including computer program instructions (as can any menu screens described in the figures). These computer program instructions may be loaded onto a computer or other programmable data processing apparatus (such as a controller, microcontroller, or processor in a sensor electronics device) to produce a machine, such that the instructions that execute on the computer or other programmable data processing apparatus create instructions for implementing the functions specified in the flowchart block or blocks. These computer program instructions may also be stored in a computer-readable memory that can direct a computer or other programmable data processing apparatus to function in a particular manner, such that the instructions stored in the computer-readable memory produce an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions which execute on the computer or other programmable apparatus provide steps for implementing the functions specified in the flowchart block or blocks, and/or menus presented herein. Programming instructions may also be stored in and/or implemented via electronic circuitry, including integrated circuits (ICs) and Application Specific Integrated Circuits (ASICs) used in conjunction with sensor devices, apparatuses, and systems.

FIG. 1 is a perspective view of simplified representation of a sensor and a block diagram of a sensor electronics device according to various aspects of the disclosure. As illustrated in FIG. 1, a sensor set 10 is provided for placement of an active portion of an analyte sensor 12, or the like, at a selected site in the body of a user. The sensor device 10 is affixed to the skin "S" by way of an adhesive patch 104, which holds the sensor device 10 in position with its analyte sensor 12 inserted into the skin "S." The sensor may be inserted into the subcutaneous tissue to sense one or more analytes in the interstitial fluid of a patient.

In particular aspects, the subcutaneous sensor set 10 facilitates the accurate placement of a flexible thin film electrochemical analyte sensor 12 of the type used for monitoring specific blood parameters representative of a user's condition. The analyte sensor 12 monitors glucose levels in the body and may be used in conjunction with automated or semi-automated medication infusion pumps of the external or implantable type to control the delivery of insulin to a diabetic patient. Examples of such infusion pumps are described, e.g., in U.S. Patent Nos. 4,562,751; 4,678,408; 4,685,903; or 4,573,994, the entire contents of each of which are incorporated herein by reference.

Particular aspects of the flexible analyte sensor 12 are constructed in accordance with thin film mask techniques to include elongated thin film conductors embedded or encased between layers of a selected insulative material such as polyimide film or sheet, and membranes. The sensor electrodes 20 at a tip end of the sensing portion 18 are exposed through one of the insulative layers for direct contact with patient blood or other body fluids, when the sensing portion 18 (or active portion) of the analyte sensor 12 is subcutaneously placed at an insertion site. The sensing portion 18 is joined to a connection portion 24 that terminates in conductive contact pads, or the like, which are also exposed through one of the insulative layers. In alternative aspects, other types of implantable sensors, such as chemical based, optical based, or the like, may be used.

The connection portion 24 and the contact pads are generally adapted for a direct wired electrical connection to a suitable monitor or sensor electronics device 100 for monitoring a user's condition in response to signals derived from the sensor electrodes 20. Further description of flexible thin film sensors of this general type may be found, e.g., in U.S. Patent No. 5,391,250, which is incorporated by reference herein in its entirety. The connection portion 24 may be conveniently connected electrically to the monitor or sensor electronics device 100 or by a connector block 28 (or the like) as shown and described, e.g., in U.S. Patent No. 5,482,473, which is also incorporated by reference herein in its entirety. Thus, in accordance with aspects of the present disclosure, subcutaneous sensor sets 10 may be configured to work with either a wired characteristic monitor system or a wireless characteristic monitor system.

The sensor electrodes 20 may be used in a variety of sensing applications and may be configured in a variety of ways. For example, the sensor electrodes 20 may be used in physiological parameter sensing applications in which some type of biomolecule is used as a catalytic agent. For example, the sensor electrodes 20 may be used in an oxygen-independent glucose sensor.

The sensor electrodes 20, along with a biomolecule or some other catalytic agent, may be placed in a human body in a vascular or non-vascular environment. For example, the sensor electrodes 20 and biomolecule may be placed in a vein and be subjected to a blood stream, or may be placed in a subcutaneous or peritoneal region of the human body.

The monitor may also be referred to as a sensor electronics device 100. The monitor may include a power source 110, a sensor interface 122, processing electronics 124, and data formatting electronics 128. The monitor may be coupled to the sensor set 10 by a cable 102 through a connector that is electrically coupled to the connector block 28 of the connection portion 24. In an alternative aspect, the cable 102 may be omitted. In this aspect of the disclosure, the monitor may include an appropriate connector for direct connection to the connection portion of the sensor set 10. The sensor set 10 may be modified to have the connector portion positioned at a different location, e.g., on top of the sensor set 10 to facilitate placement of the monitor over the sensor set 10.

In aspects of the disclosure, the sensor interface 122, the processing electronics 124, and the data formatting electronics 128 are formed as separate semiconductor chips, however, alternative aspects may combine the various semiconductor chips into a single or multiple customized semiconductor chips. The sensor interface 122 connects with the cable 102 that is connected with the sensor set 10.

The power source 110 may be a battery. The battery can include three series silver oxide battery cells. In alternative aspects, different battery chemistries may be utilized, such as lithium based chemistries, alkaline batteries, nickel metal hydride, or the like, and a different number of batteries may be used. The monitor 100 provides power to the sensor set via the power source 110, through the cable 102 and cable connector. In an aspect of the disclosure, the power is a voltage provided to the sensor set 10. In an aspect of the disclosure, the power is a current provided to the sensor set 10. In an aspect of the disclosure, the power is a voltage provided at a specific voltage to the sensor set 10.

FIG. 2 illustrates a general block diagram of an electronic circuit for sensing the output of an analyte sensor according to aspects of the present disclosure. At least one pair of sensor electrodes 310 may interface to a data converter 312, the output of which may interface to a counter 314. The counter 314 may be controlled by control logic 316. The output of the counter 314 may connect to a line interface 318. The line interface 318 may be connected to input and output lines 320 and may also connect to the control logic 316. The input and output lines 320 may also be connected to a power rectifier 322.

FIG. 3 illustrates a block diagram of an analyte sensor electronics device and a sensor including a plurality of electrodes according to an aspect of the disclosure. The sensor set or system 350 includes an analyte sensor 355 and a sensor electronics device 360. The analyte sensor 355 includes a counter electrode 365, a reference electrode 370, and a working electrode 375. The sensor electronics device 360 includes a power supply 380, a regulator 385, a signal processor 390, a measurement processor 395, and a display/transmission module 397. The power supply 380 provides power (in the form of either a voltage, a current, or a voltage including a current) to the regulator 385. The regulator 385 transmits a

regulated voltage to the analyte sensor 355. In an aspect of the disclosure, the regulator 385 transmits a voltage to the counter electrode 365 of the analyte sensor 355.

The analyte sensor 355 creates a sensor signal indicative of a concentration of a physiological characteristic being measured. For example, the sensor signal may be indicative of a blood glucose reading. The sensor signal may be measured at the working electrode 375. In an aspect of the disclosure, the sensor signal may be a current measured at the working electrode. In an aspect of the disclosure, the sensor signal may be a voltage measured at the working electrode.

The signal processor 390 receives the sensor signal (e.g., a measured current or voltage) after the sensor signal is measured at the analyte sensor 355 (e.g., the working electrode). The signal processor 390 processes the sensor signal and generates a processed sensor signal. The measurement processor 395 receives the processed sensor signal and calibrates the processed sensor signal utilizing reference values. In an aspect of the disclosure, the reference values are stored in a reference memory and provided to the measurement processor 395. The measurement processor 395 generates sensor measurements. The sensor measurements may be stored in a measurement memory (not shown). The sensor measurements may be sent to a display/transmission device to be either displayed on a display in a housing with the sensor electronics or transmitted to an external device.

The sensor electronics device 360 may be a monitor that includes a display to display physiological characteristics readings. The sensor electronics device 360 may also be installed in a desktop computer, a pager, a television including communications capabilities, a laptop computer, a server, a network computer, a personal digital assistant (PDA), a portable telephone including computer functions, an infusion pump including a display, and/or a combination infusion pump/ analyte sensor. The sensor electronics device 360 may be housed in a cellular phone, a smartphone, a network device, a home network device, and/or other appliances connected to a home network.

FIG. 4 illustrates an analyte sensor and a sensor electronics device according to an alternative aspect of the present disclosure. The sensor set or sensor system 400 includes the sensor electronics device 360 and the analyte sensor 355. The analyte sensor 355 includes the counter electrode 365, the reference electrode 370, and the working electrode 375. The sensor electronics device 360 includes a microcontroller 410 and a digital-to-analog converter (DAC) 420. The sensor electronics device 360 may also include a current-to-frequency converter (I/F converter) 430.

The microcontroller 410 includes software program code or programmable logic which, when executed, causes the microcontroller 410 to transmit a signal to the DAC 420, where the signal is representative of a voltage level or value that is to be applied to the analyte sensor 355. The DAC 420 receives the signal and generates the voltage value at the level instructed by the microcontroller 410. In aspects of the disclosure, the microcontroller 410 may change the representation of the voltage level in the signal frequently or infrequently. Illustratively, the signal from the microcontroller 410 may instruct the DAC 420 to apply a first voltage value for one second and a second voltage value for two seconds.

The analyte sensor 355 may receive the voltage level or value. In an aspect of the disclosure, the counter electrode 365 may receive the output of an operational amplifier which has as inputs the reference voltage and the voltage value from the DAC 420. The application of the voltage level causes the analyte sensor 355 to create a sensor signal indicative of a concentration of a physiological characteristic being measured. In an aspect of the disclosure, the microcontroller 410 may measure the sensor signal (e.g., a current value) from the working electrode. Illustratively, a sensor signal measurement circuit 431 may measure the sensor signal. In an aspect of the disclosure, the sensor signal measurement circuit 431 may include a resistor and the current may be passed through the resistor to measure the value of the sensor signal. In an aspect of the disclosure, the sensor signal may be a current level signal and the sensor signal measurement circuit 431 may be a current-to-frequency (I/F) converter 430. The I/F converter 430 may measure the sensor signal in terms of a current reading, convert it to a frequency-based sensor signal or electrochemical impedance spectroscopy ("EIS") signal, and transmit the frequency-based sensor signal or EIS signal to the microcontroller 410. Persons skilled in the art will understand how to implement and apply EIS. Various aspects of EIS signals are described in U.S. Patent No. 9,642,568, which is hereby incorporated by reference herein in its entirety. In aspects of the disclosure, the microcontroller 410 may be able to receive frequency-based sensor signals easier than non-frequency-based sensor signals. The microcontroller 410 receives the sensor signal, whether frequency-based or non-frequency-based, and determines a value for the physiological characteristic of a subject, such as a blood glucose level. The microcontroller 410 may include program code, which when executed, is able to receive the sensor signal and convert the sensor signal to a physiological characteristic value.

In one aspect of the disclosure, the microcontroller 410 may convert the sensor signal to a blood glucose value. While converting the sensor signal to a blood glucose value, the microcontroller 410 may use one or more models, which are specific ways to use the sensor signal to calculate the blood glucose value. In some aspects, the microcontroller 410 may utilize measurements (e.g., sensor signals and electrochemical impedance spectroscopy (EIS) signals from the analyte sensor 355) stored within an internal memory in order to determine the blood glucose level of the subject. In some aspects, the microcontroller 410 may utilize measurements stored within a memory external to the microcontroller 410 to assist in determining the blood glucose value of the subject.

After the physiological characteristic value is determined by the microcontroller 410, the microcontroller 410 may store measurements of the physiological characteristic values for a number of time periods. For example, a blood glucose

value (BG) may be sent to the microcontroller 410 from the sensor every second or every five seconds, and the microcontroller may save sensor measurements for five minutes or ten minutes of BG readings. The microcontroller 410 may transfer the measurements of the physiological characteristic values to a display on the sensor electronics device 360. For example, the sensor electronics device 360 may be a monitor which includes a display that provides a blood glucose reading for a subject. In one aspect of the disclosure, the microcontroller 410 may transfer the measurements of the physiological characteristic values to an output interface of the microcontroller 410. The output interface of the microcontroller 410 may transfer the measurements of the physiological characteristic values, e.g., blood glucose values, to an external device, e.g., an infusion pump, a combined infusion pump/glucose meter, a computer, a personal digital assistant, a pager, a network appliance, a server, a cellular phone, or any computing device.

FIG. 5 is a graph illustrating two sensor signals (iSig) over time from two respective working electrodes of an analyte sensor that have different electrocatalytic activities. It is beneficial for continuous glucose monitoring (CGM) to have a quick start to reach a functional state for on-adjunctive use (e.g., minimize warm-up/run-in time and stabilize Day 1 performance). The disclosed technology enables the use of two or more different working electrodes on the same analyte sensor, with differing electrocatalytic activity, to minimize warm-up/run-in time of the analyte sensor.

Working electrodes with differing surface area roughness will have differing longevity and warm-up/run-in performance, as is evidenced by the traces in FIG. 5. The trace for the second sensor signal 604 over time, for a working electrode that has a lower surface area roughness, demonstrates that this working electrode has a shorter life but also has a faster run-in than the first sensor signal 602, which has a higher surface area roughness. There is a region of the graph where the sensor signals 602 and 604 may perform similarly and this region may be used as a handover region 606. FIG. 6 is a graph illustrating a fused sensor signal 702 based on the signals of FIG. 5. The fusing of the first sensor signal 602 and the second sensor signal 604 to form the fused sensor signal 702 is described in detail below (FIG. 10). The fused sensor signal serves to improve run-in time, interferent rejection, and longevity of the analyte sensor 800, 900, 1000 (FIGS. 7-9).

FIG. 7 is a diagram of an example analyte sensor 800 of the device of FIG. 1 having two working electrodes that each have different electrocatalytic activity. The analyte sensor 800 generally includes a substrate 802 (e.g., a sensor flex), a counter electrode (CE) 804, a reference electrode 806, a first working electrode (WE1) 810, and a second working electrode (WE2) 808.

The working electrodes generally include a stack (e.g., a layering or arrangement of different materials configured to achieve specific functionality). For example, the stack may include, among other materials, gold, carbon, platinum (Pt) or platinum iridium. The first working electrode 810 includes an analyte sensing molecule, disposed on the first working electrode, that is configured to generate a first sensor signal (e.g., a current) when exposed to an analyte (e.g., glucose). The second working electrode 808 includes an analyte sensing molecule disposed on the second working electrode that is configured to generate a second sensor signal (e.g., a current), different from the first sensor signal when exposed to the analyte.

The two working electrodes are configured to each have a different electrocatalytic activity. The first working electrode 810 and the second working electrode 808 may include, among other materials, platinum. In aspects, the first working electrode 810 may have a high surface area roughness (e.g., a ratio of Pt roughness compared to the geometric area of about 15 to about 400). In aspects, the second working electrode 808 may have a low surface area roughness (e.g., a ratio of Pt roughness compared to the geometric area of about 2 to about 15). In aspects, the working electrode geometric area of each working electrode 808, 810 may be scaled such that the sensor signal (e.g., iSig) generated by each working electrode may be equal when exposed to a given glucose value. In order to accomplish this, the working electrode with the lower surface area may have a larger geometric area relative to the working electrode with the higher surface area.

Since the iSig does not scale 1: 1 with surface area roughness, the following condition will need to be met:

$$\frac{WE_{2,Geometric\ Area}}{WE_{1,Geometric\ Area}} > \frac{WE_{2,Surface\ Area}}{WE_{1,SurfaceArea}}.$$

The working electrode with the higher surface area roughness (which may be used for late wear measurements) is placed more distally on the sensor flex relative to the electrode that is used for early wear measurements. The reason for this is that previous in-vivo studies demonstrated better sensor longevity with a working electrode placed deeper in the tissue. This approach provides the advantage that the iSig on the first working electrode 810 will be more closely matched to the second working electrode 808 in-vivo at the time that the algorithm transitions from the first working electrode 810 signal to the second working electrode 808 sensor signal, which will reduce the burden on any given algorithm to determine the appropriate scaling factor to apply to the raw iSig of the first working electrode 810 or the second working electrode 808.

FIG. 8 is a diagram illustrating another example analyte sensor 900 of the device of FIG. 1, having two working electrodes that have different electrocatalytic activities and, one of which, also has an interference rejection membrane (IRM). The presence of an IRM often slows down the warm-up process for an analyte sensor. The analyte sensor 900 generally includes a substrate 902 (e.g., a sensor flex), a counter electrode (CE) 904, a reference electrode 906, a first working electrode (WE1) 910 that includes the IRM, and a second working electrode (WE2) 908 that does not include an IRM. Thus, the layer stack on one working electrode includes an IRM, while the other one does not. In aspects, the IRM

layer is added to the electrode with higher roughness. However, it is contemplated that the IRM layer may be added to the electrode with lower roughness. The IRM layer provides the benefit of enabling the suitability of all days during the wear-time for AC mode intake without counter-labelling. AC mode is a mode on some devices that enables users to check their fasting sugar before breakfast.

In aspects of the disclosure, IRM may be added to the first working electrode 910 only (e.g., the working electrode that has a high surface area roughness, which is configured to generate the sensor signal to be used in the later stages of the wear). The second working electrode 908 (e.g., with low surface area roughness) may be without the interference rejection membrane.

FIG. 9 is a diagram illustrating another example analyte sensor 1000 of the device of FIG. 1, which has two different interference rejection membranes. The analyte sensor 1000 generally includes a substrate 1002 (e.g., a sensor flex), a counter electrode (CE) 1004, a reference electrode 1006, a first working electrode (WE1) 1010 that includes a first IRM, and a second working electrode (WE2) 1008 that includes a second IRM different from the first IRM. For example, one IRM may be crosslinked for providing relatively faster run-in time than the other IRM. In another example, one IRM may be crosslinked for providing longevity but not for providing faster run-in time.

For example, the IRM may include 5 wt % poly(vinyl alcohol) crosslinked with 10-20 wt % crosslinker. Typically, the poly(vinyl alcohol) polymer has a molecular weight (Mw) of at least 45K. In further instances, the degree of hydrolysis of the poly(vinyl alcohol) polymer is at least 98%. In aspects, the crosslinker is selected from the group consisting of sulfosuccinic acid (SSA), maleic acid, citric acid, oxalic acid, fumaric acid, poly(acrylic acid), poly(acrylic acid-co-maleic acid) (PAM), succinic acid, malonic acid, and poly(methyl vinyl ether-alt-maleic acid). In one instance, the crosslinker is sulfosuccinic acid. In certain embodiments, the interference rej ection membrane comprises 5-50 wt % sulfosuccinic acid (based on the polymer weight of PVA in solution). In one illustrative implementation, the interference rejection membrane comprises 5 wt % poly(vinyl alcohol) crosslinked with 10 wt % sulfosuccinic acid. In another instance, the crosslinker is poly(methyl vinyl ether-alt-maleic acid).

FIG. 10 is a flow diagram for processor-implemented method 1100 of compensating for analyte interference for the analyte sensor 12 of FIG. 1. The method 1100 may be implemented by sensor electronics device 100 of FIG. 1, sensor electronics device 360 of FIGS. 3 and 4, or analyte sensors 800, 900, 1000 of FIGS. 7-9.

Generally, the processor applies a bias voltage to the working electrode of the analyte sensor 800, 900, and 1000 to adjust the sensitivity of a measurement of an analyte. The bias voltage is generally within a range of insulin excipient oxidation (e.g., about 300 to about 600mV). This can cause the delivery of a medication bolus (e.g., insulin) to interfere with the accuracy of the analyte sensor's measurements.

In an example, the analyte sensor 800 (FIG. 7) may have a first working electrode 810 and a second working electrode 808. The second working electrode 808 is configured to generate a second sensor signal, different from a first sensor signal generated by the first working electrode 810 when exposed to the analyte. The Pt portions of each working electrode have a surface area roughness. For example, the first working electrode may have a first Pt roughness and the second working electrode may have a second Pt different from the first Pt roughness. The second working electrode 808 may have a different electrocatalytic activity than the first working electrode 810. In aspects, the first working electrode 810 and the second working electrode 808 may be operated independently. In aspects, the first working electrode and the second working electrode have a different operating potential than each other.

At block 1102, the processor causes the analyte sensor 800 to measure the first sensor signal 602 at the first working electrode 810. At block 1104, the processor causes the analyte sensor 800 to measure the second sensor 604 signal at the second working electrode. For example, the processor may cause the analyte sensor to obtain the signals 602, 604 from the working electrodes 808, 810, each indicating a respective analyte value (FIG. 5).

In aspects, the processor may cause the sensor 12 to generate an electrochemical impedance spectroscopy (EIS) parameter value and/or a conductivity value in response to exposure of the working electrodes to the analyte. In aspects, the processor determines the presence of an interferent (e.g., insulin) based on at least one of the first EIS parameter values or the first conductivity value. An interferent is a substance that interferes with an analytical procedure resulting in incorrect results. The EIS value may be determined for each working electrode sensor signal or may be determined based on a fused sensor signal, which is described in detail below.

At block 1106, the processor determines a hand-off period for the sensor. In aspects, determining the hand-off period may be based on a rate of change of the sensor glucose of the first working electrode being below a predetermined threshold. For example, the second sensor signal may be of a lower value than the first sensor signal.

In aspects, the hand-off period (handover region 606 FIG. 5) can happen at a predetermined time for all sensors. For example, in a case where sensitivity loss occurs constantly across all sensors by about day six for a shiny Pt (low surface area roughness) working electrode, and a high surface area roughness Pt working electrode has fully stabilized after about twelve hours, then the hand-off can be configured to happen at a time: $t_{Hand\text{-}off}$, such that 12hr < $t_{Hand\text{-}off}$< Day 6. For example, the hand-off can occur at a unique time for each sensor, which is determined in real-time by the processor when certain criteria are met (e.g., when it is determined that the sensor glucose of a high surface area roughness Pt working electrode has stabilized).

In an aspect, the processor may bias a weight on one sensor signal (e.g., the first sensor signal) over the other sensor signal (e.g., the second sensor signal). This may enable the processor to use the less biased signal for sensor quality checks. In aspects, the processor may track a calibration factor and assign weight to different traces for a composite signal. A calibration factor represents a sensor sensitivity for a particular sensor. The calibration factor is equal to the blood glucose/(iSig-offset). In aspects, the offset may be a predetermined value. In aspects, the processor may use the lower weighted sensor signal to perform sensor health diagnostics (e.g., to check for noise profiles and/or compare the two sensor signals).

In aspects, the processor may use the better-quality trace during the wear and could be multiple hand-offs during wear. The better-quality sensor signal (e.g., the stabilized signal) of the two sensor signals as used herein includes the sensor signal that has stabilized over time. In aspects, the processor may use other parameters such as EIS signatures to determine when a hand-off should occur.

At block 1108, the processor generates a fused sensor signal based on using the first sensor signal for a first period of time and after the hand-off period switching to using the second sensor signal for a second period of time (FIG. 6).

In aspects, the processor may cause the sensor, in response to the delivery of the bolus, to obtain the signal from the working electrode. The signal may be processed to generate an EIS parameter value and/or a conductivity value. In aspects, the processor may use the second working electrode to generate $O_2$ into the local environment to maintain stable $O_2$ levels.

In aspects, the processor may use a second electrode with a different electrochemical technique to enable the differentiation of acetaminophen vs glucose. In aspects, the processor may quantify tissue environment (e.g., foreign body response "FBR") at different bias voltages (vSet).

In aspects, the processor may track the performance of the individual WEs and transition from one WE to another in mid-wear. For example, the processor may track the performance of the first working electrode and the second working electrode. The processor may determine a wear profile based on the tracking, and generate the fused sensor signal based on a mid-wear point in the wear profile of each working electrode.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, the above-described servers and computing devices.

While the description above refers to particular aspects of the present disclosure, it will be understood that many modifications may be made without departing from the spirit thereof. Additional steps and changes to the order of the algorithms can be made while still performing the key teachings of the present disclosure. Thus, the accompanying claims are intended to cover such modifications as would fall within the true scope and spirit of the present disclosure. The presently disclosed aspects are, therefore, to be considered in all respects as illustrative and not restrictive, the scope of the disclosure being indicated by the appended claims rather than the foregoing description. Unless the context indicates otherwise, any aspect disclosed herein may be combined with any other aspect or aspects disclosed herein. All changes that come within the meaning of, and range of, equivalency of the claims are intended to be embraced therein.

Further disclosed herein is the subject-matter of the following clauses:

1. An analyte sensor, comprising:

   a first working electrode including an analyte sensing molecule disposed on the first working electrode and configured to generate a first sensor signal when exposed to an analyte;
   a second working electrode including an analyte sensing molecule disposed on the second working electrode and configured to generate a second sensor signal, different from the first sensor signal, when exposed to an analyte, wherein the first and second working electrodes each have different electrocatalytic activity;
   a processor; and
   a memory, including instructions which, when executed by the processor, cause the analyte sensor to:

      measure the first sensor signal at the first working electrode;
      measure the second sensor signal at the second working electrode;
      determine a hand-off period for the sensor; and
      generate a fused sensor signal based on the first sensor signal for a first period of time and after the hand-off period, based on the second sensor signal for a second period of time.

2. The analyte sensor of clause 1, wherein the first working electrode has a first platinum roughness, and the second working electrode has a second platinum roughness different than the first platinum roughness.

3. The analyte sensor of clause 1 or 2, wherein the first working electrode and the second working electrode have at least one layer of a stack that differs between them.

4. The analyte sensor of clause 1 or of one of clauses 1 to 3, wherein the first working electrode and the second working electrode are operated independently and wherein first working electrode and the second working electrode have a different operating potential than each other.

5. The analyte sensor of clause 1 or of one of clauses 1 to 4, wherein a geometric area of each working electrode is scaled such that a sensor signal generated on each work electrode is equal when exposed to a given analyte value.

6. The analyte sensor of clause 1 or of one of clauses 1 to 5, wherein the hand-off period is a predetermined period of time.

7. The analyte sensor of clause 1 or of one of clauses 1 to 6, wherein the instructions, when executed by the processor, further cause the analyte sensor to:

determine a first sensor glucose value based on the first sensor signal; and
determine a second sensor glucose value based on the second sensor signal,
wherein determining the hand-off period is based on a rate of change of the first sensor glucose value of the first working electrode being below a predetermined threshold.

8. The analyte sensor of clause 1 or of one of clauses 1 to 7, wherein one of the first or second working electrodes has a higher surface area roughness than the other of the first or second working electrodes, and the one of the first or second working electrodes with the higher surface area roughness is located more distally on the sensor relative to the other of the first or second working electrodes.

9. The analyte sensor of clause 1 or of one of clauses 1 to 8, wherein the instructions, when executed by the processor, further cause the analyte sensor to:
determine at least one of an electrochemical impedance spectroscopy (EIS) parameter value or a conductivity value based on the fused sensor signal.

10. The analyte sensor of clause 1 or of one of clauses 1 to 9, wherein the sensor includes an interference rejection membrane on at least one of the first working electrode or the second working electrode.

11. A processor-implemented method for operating an analyte sensor, the method comprising:

measuring a first sensor signal at a first working electrode, the first working electrode including an analyte sensing molecule disposed on the first working electrode and configured to generate a first signal when exposed to an analyte;
measuring a second sensor signal at a second working electrode, the second working electrode including an analyte sensing molecule disposed on the second working electrode and configured to generate a second signal when exposed to an analyte;
determining a hand-off period for the analyte sensor; and
generating a fused sensor signal based on the first sensor signal for a first period of time and after the hand-off period, based on the second sensor signal for a second period of time.

12. The processor-implemented method of clause 11, further comprising:

determining a first sensor glucose value based on the first sensor signal,
wherein determining the hand-off period is based on a rate of change of the first sensor glucose value of the first working electrode being below a predetermined threshold.

13. The processor-implemented method of clause 11 or 12, further comprising:

tracking performance of the first working electrode and the second working electrode;

determining a wear profile based on the tracking; and
generating the fused sensor signal based on a mid-wear point in the wear profile of each working electrode.

14. The processor-implemented method of clause 11 or of one of clauses 11 to 13, further comprising:
independently operating the first working electrode and the second working electrode.

15. The processor-implemented method of clause 11 or of one of clauses 11 to 14,
wherein the second working electrode has a different electrocatalytic activity than the first working electrode.

16. The processor-implemented method of clause 11 or of one of clauses 11 to 15,
wherein determining the hand-off period for the analyte sensor comprises setting the hand-off period to a predetermined period of time.

17. The processor-implemented method of clause 11 or of one of clauses 11 to 16,
wherein the first working electrode has a first platinum roughness and the second working electrode has a second platinum roughness different than the first platinum roughness.

18. The processor-implemented method of clause 11 or of one of clauses 11 to 17, further comprising:
determining at least one of an electrochemical impedance spectroscopy (EIS) parameter value or a conductivity value based on the fused sensor signal.

19. The processor-implemented method of clause 18, further comprising:
determining a presence of an interferent based on at least one of the EIS parameter value or the conductivity value.

20. One or more non-transitory processor-readable media storing instructions which, when executed by one or more processors, cause performance of:

measuring a first sensor signal at a first working electrode of an analyte sensor, the first working electrode including an analyte sensing molecule disposed on the first working electrode and configured to generate a first signal when exposed to an analyte;
measuring a second sensor signal at a second working electrode of the analyte sensor, the second working electrode including an analyte sensing molecule disposed on the second working electrode and configured to generate a second signal when exposed to an analyte;
determining a hand-off period for the analyte sensor; and
generating a fused sensor signal based on the first sensor signal for a first period of time and, after the hand-off period, based on the second sensor signal for a second period of time.

## Claims

**1.** An analyte sensor, comprising:

a first working electrode including an analyte sensing molecule disposed on the first working electrode and configured to generate a first sensor signal when exposed to an analyte;
a second working electrode including an analyte sensing molecule disposed on the second working electrode and configured to generate a second sensor signal, different from the first sensor signal, when exposed to an analyte, wherein the first and second working electrodes each have different electrocatalytic activity;
a processor; and
a memory, including instructions which, when executed by the processor, cause the analyte sensor to:

measure the first sensor signal at the first working electrode;
measure the second sensor signal at the second working electrode;
determine a hand-off period for the sensor; and
generate a fused sensor signal based on the first sensor signal for a first period of time and after the hand-off period, based on the second sensor signal for a second period of time.

**2.** The analyte sensor of claim **1,** wherein the first working electrode has a first platinum roughness, and the second working electrode has a second platinum roughness different than the first platinum roughness,

and/or

wherein one of the first or second working electrodes has a higher surface area roughness than the other of the first or second working electrodes, and the one of the first or second working electrodes with the higher surface area roughness is located more distally on the sensor relative to the other of the first or second working electrodes.

**3.** The analyte sensor of claim 1 or 2, wherein the first working electrode and the second working electrode have at least one layer of a stack that differs between them,

and/or

wherein the sensor includes an interference rejection membrane on at least one of the first working electrode or the second working electrode.

**4.** The analyte sensor of one of claims 1 to 3, wherein the first working electrode and the second working electrode are operated independently and wherein first working electrode and the second working electrode have a different operating potential than each other.

**5.** The analyte sensor of one of claims 1 to 4, wherein a geometric area of each working electrode is scaled such that a sensor signal generated on each work electrode is equal when exposed to a given analyte value.

**6.** The analyte sensor of one of claims 1 to 5, wherein the hand-off period is a predetermined period of time,

and/or

wherein the instructions, when executed by the processor, further cause the analyte sensor to:

determine a first sensor glucose value based on the first sensor signal; and

determine a second sensor glucose value based on the second sensor signal,

wherein determining the hand-off period is based on a rate of change of the first sensor glucose value of the first working electrode being below a predetermined threshold.

**7.** The analyte sensor of one of claims 1 to 6, wherein the instructions, when executed by the processor, further cause the analyte sensor to:

determine at least one of an electrochemical impedance spectroscopy (EIS) parameter value or a conductivity value based on the fused sensor signal.

**8.** A processor-implemented method for operating an analyte sensor, the method comprising:

measuring a first sensor signal at a first working electrode, the first working electrode including an analyte sensing molecule disposed on the first working electrode and configured to generate a first signal when exposed to an analyte;

measuring a second sensor signal at a second working electrode, the second working electrode including an analyte sensing molecule disposed on the second working electrode and configured to generate a second signal when exposed to an analyte;

determining a hand-off period for the analyte sensor; and

generating a fused sensor signal based on the first sensor signal for a first period of time and after the hand-off period, based on the second sensor signal for a second period of time.

**9.** The processor-implemented method of claim 8, further comprising:

determining a first sensor glucose value based on the first sensor signal,

wherein determining the hand-off period is based on a rate of change of the first sensor glucose value of the first working electrode being below a predetermined threshold,

and/or

wherein determining the hand-off period for the analyte sensor comprises setting the hand-off period to a predetermined period of time.

**10.** The processor-implemented method of claim 8 or 9, further comprising:

tracking performance of the first working electrode and the second working electrode;

determining a wear profile based on the tracking; and

generating the fused sensor signal based on a mid-wear point in the wear profile of each working electrode.

**11.** The processor-implemented method of one of claims 8 to 10, further comprising:
independently operating the first working electrode and the second working electrode.

**12.** The processor-implemented method of one of claims 8 to 11, wherein the second working electrode has a different electrocatalytic activity than the first working electrode.

**13.** The processor-implemented method of one of claims 8 to 12, wherein the first working electrode has a first platinum roughness and the second working electrode has a second platinum roughness different than the first platinum roughness.

**14.** The processor-implemented method of one of claims 8 to 13, further comprising:

determining at least one of an electrochemical impedance spectroscopy (EIS) parameter value or a conductivity value based on the fused sensor signal,
particularly
further comprising:
determining a presence of an interferent based on at least one of the EIS parameter value or the conductivity value.

**15.** One or more non-transitory processor-readable media storing instructions which, when executed by one or more processors, cause performance of:

measuring a first sensor signal at a first working electrode of an analyte sensor, the first working electrode including an analyte sensing molecule disposed on the first working electrode and configured to generate a first signal when exposed to an analyte;
measuring a second sensor signal at a second working electrode of the analyte sensor, the second working electrode including an analyte sensing molecule disposed on the second working electrode and configured to generate a second signal when exposed to an analyte;
determining a hand-off period for the analyte sensor; and
generating a fused sensor signal based on the first sensor signal for a first period of time and, after the hand-off period, based on the second sensor signal for a second period of time.

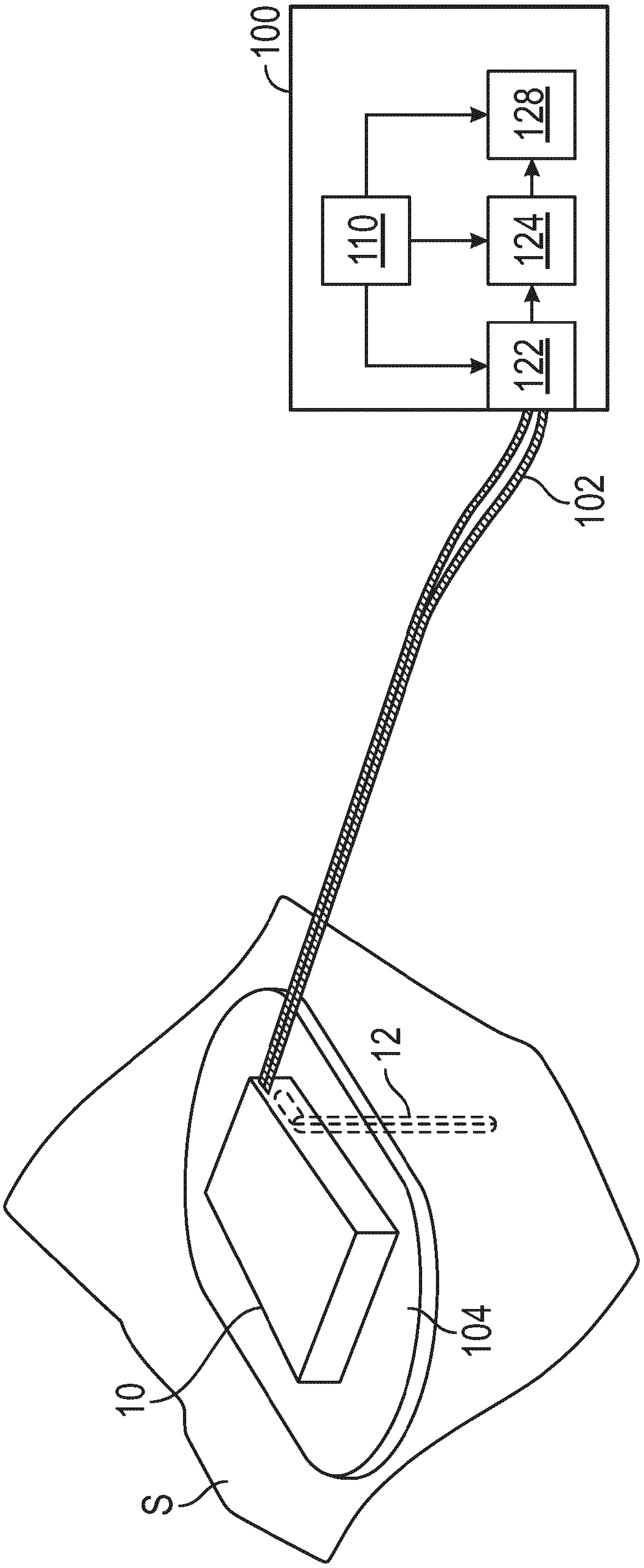


FIG. 1

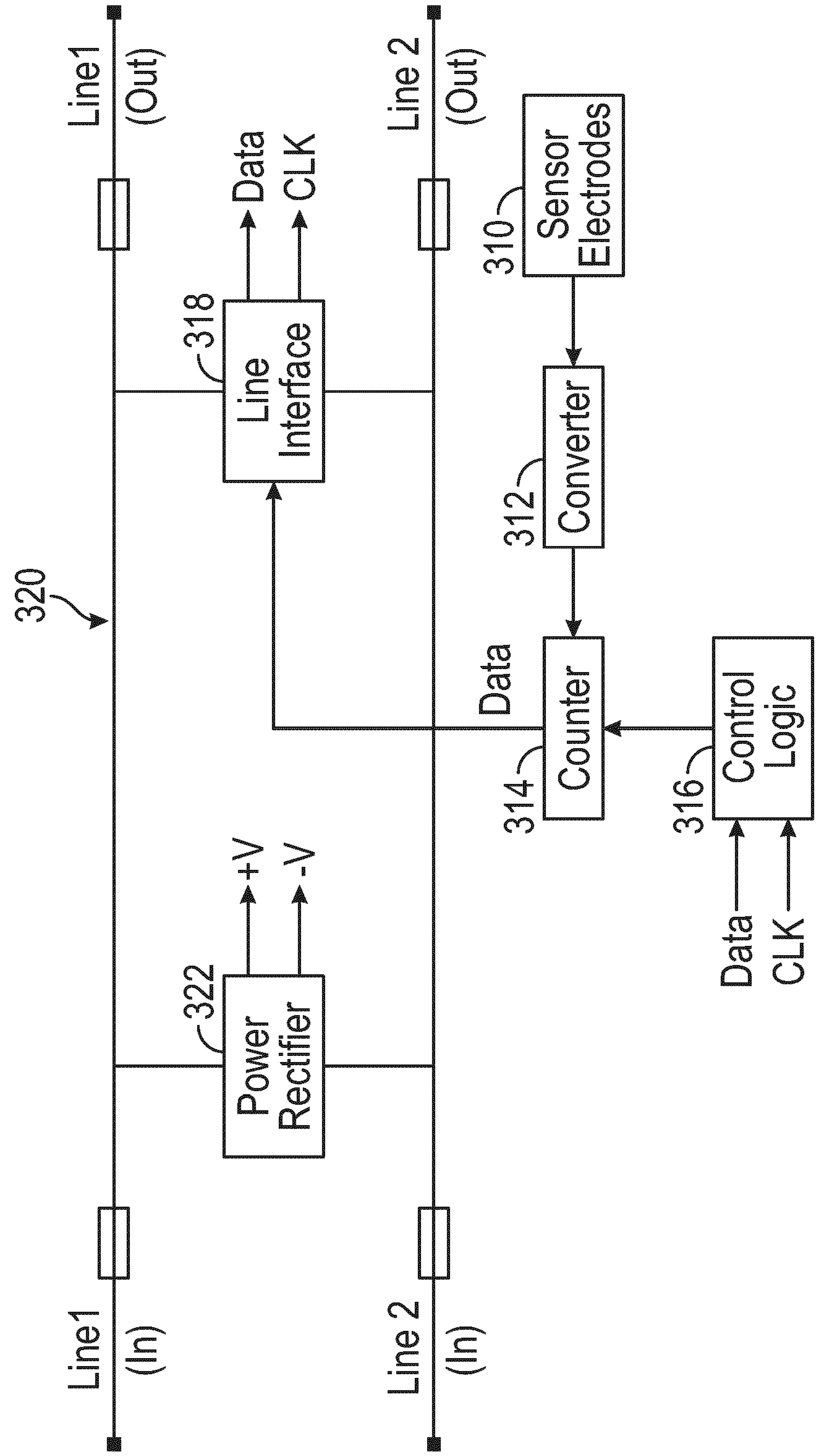
Line1 (Out)
Line 2 (Out)
Data
CLK
318
Line Interface
310
Sensor Electrodes
312
Converter
320
Data
314
Counter
316
Control Logic
Data
CLK
+V
-V
322
Power Rectifier
Line1 (In)
Line 2 (In)

FIG. 2

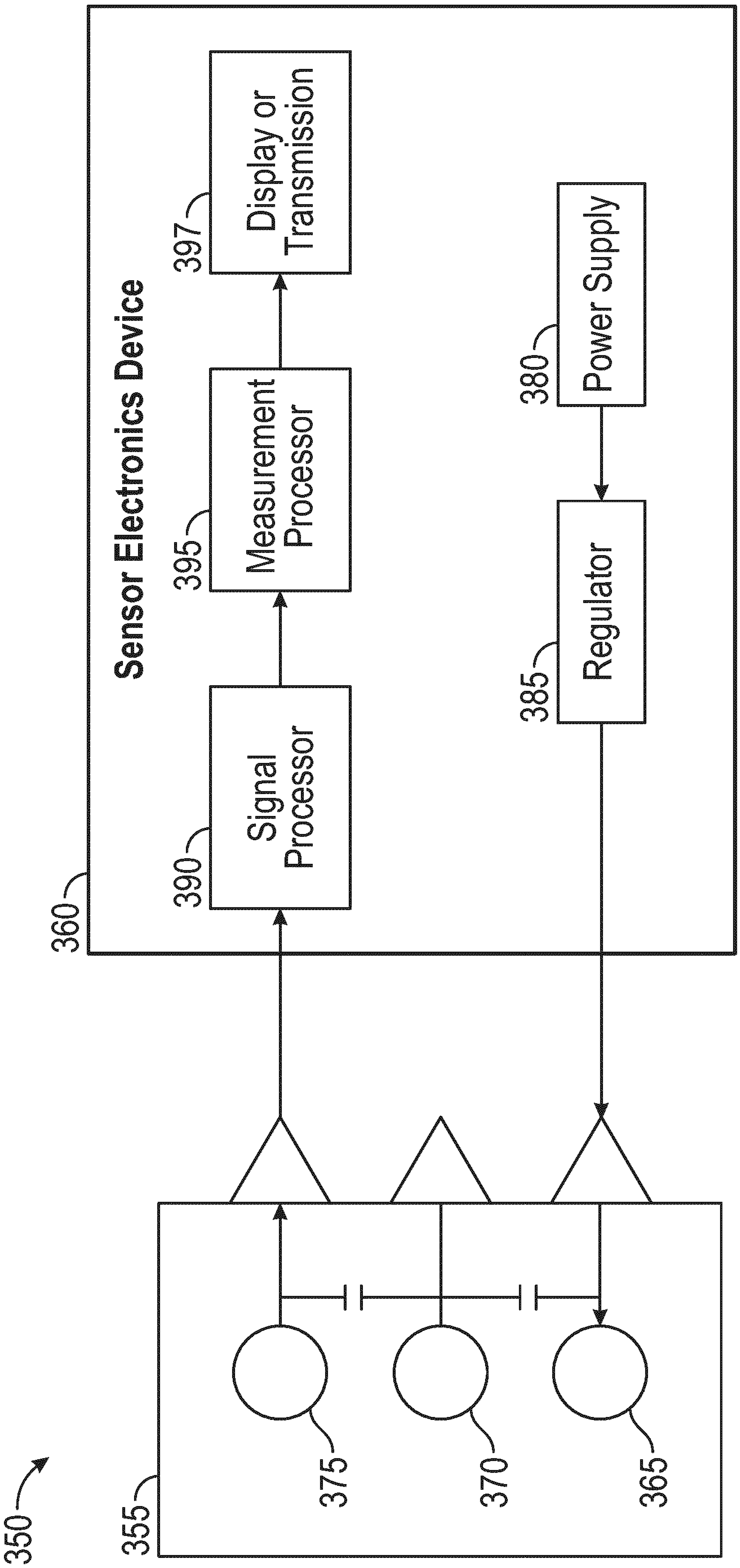
Sensor Electronics Device
Display or Transmission
Measurement Processor
Signal Processor
Power Supply
Regulator
397
395
390
380
385
360
350
355
375
370
365

FIG. 3

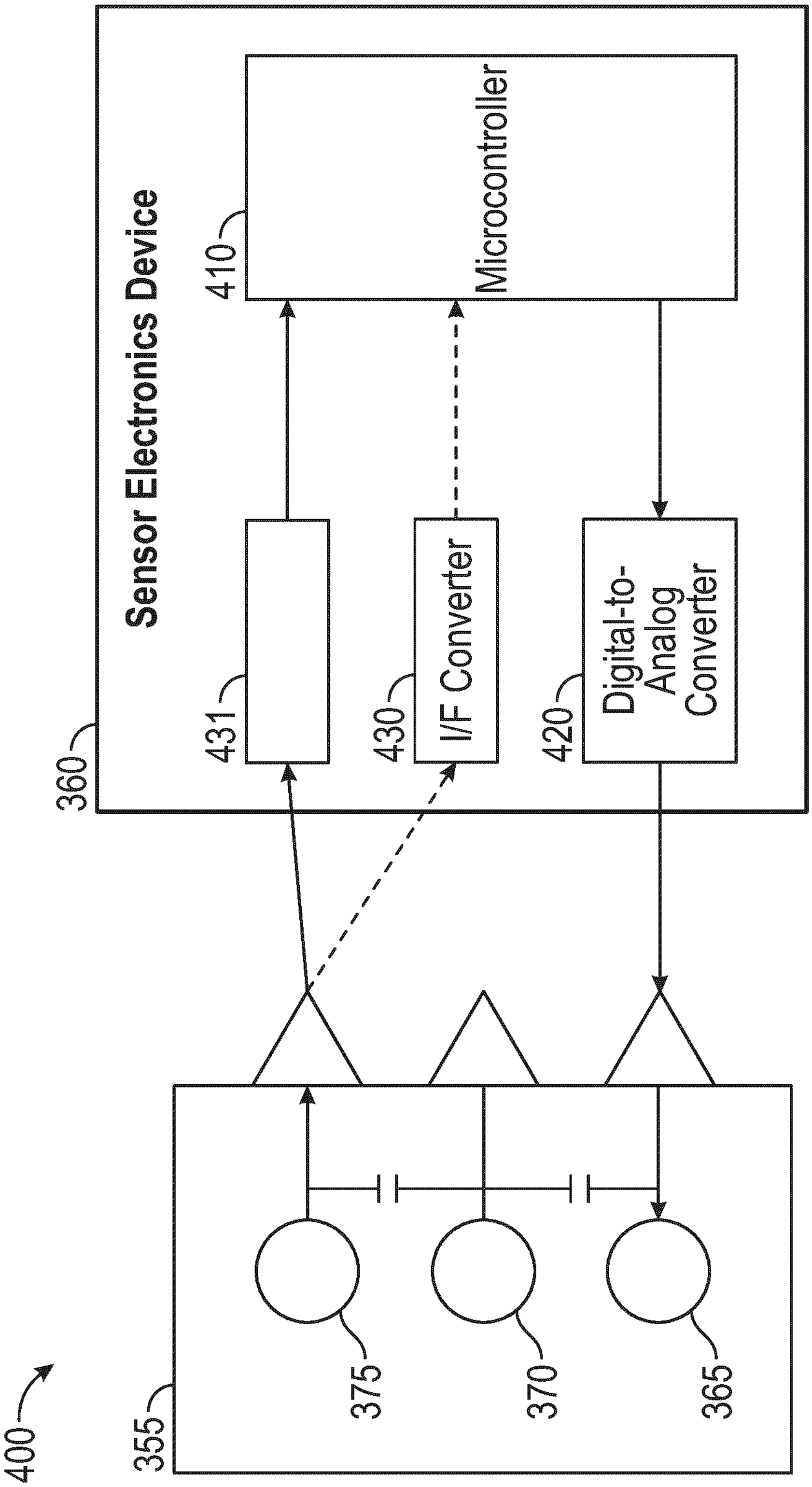
400
355
375
370
365
360
Sensor Electronics Device
431
430
I/F Converter
420
Digital-to-Analog Converter
410
Microcontroller

FIG. 4

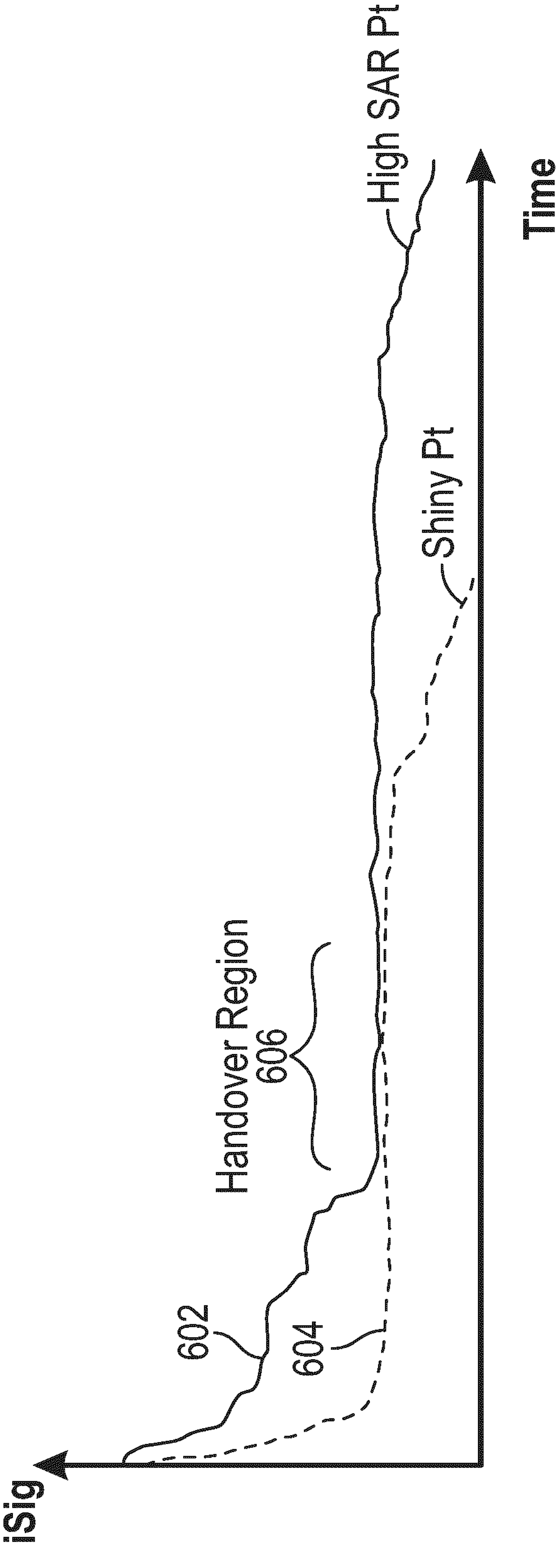
iSig
Time
High SAR Pt
Shiny Pt
Handover Region
606
602
604

FIG. 5

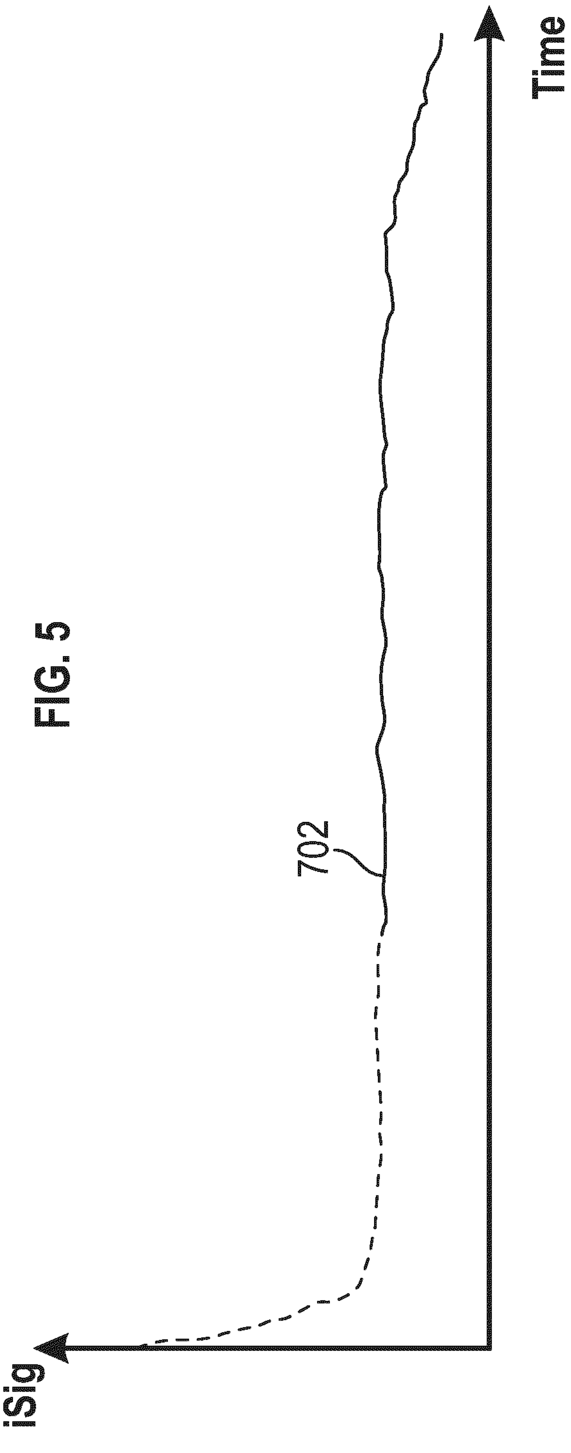
iSig
Time
702

FIG. 6

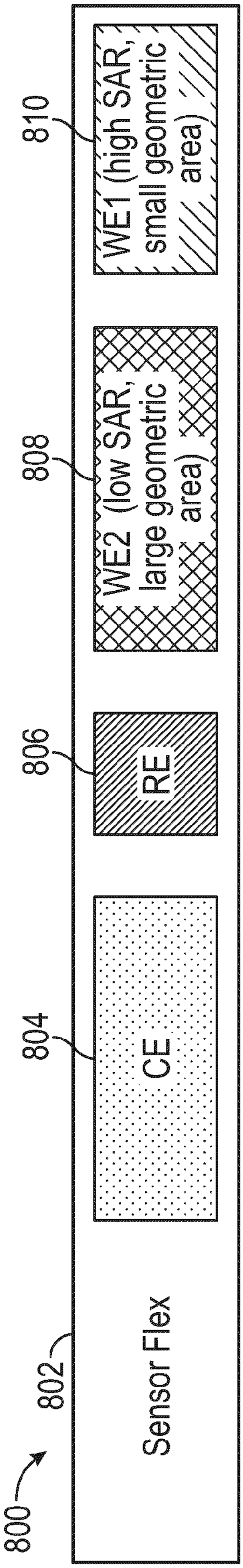


FIG. 7

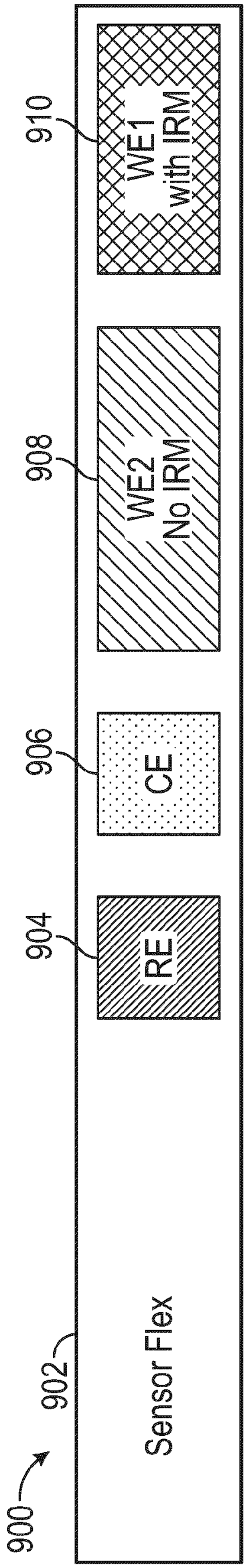


FIG. 8

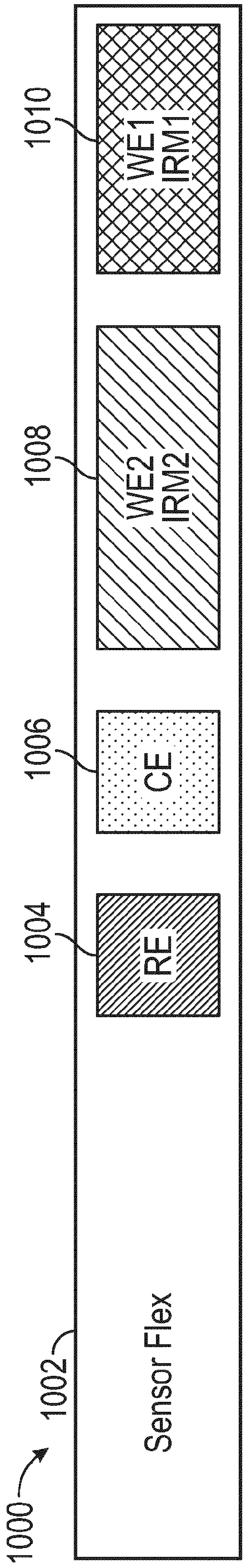


FIG. 9

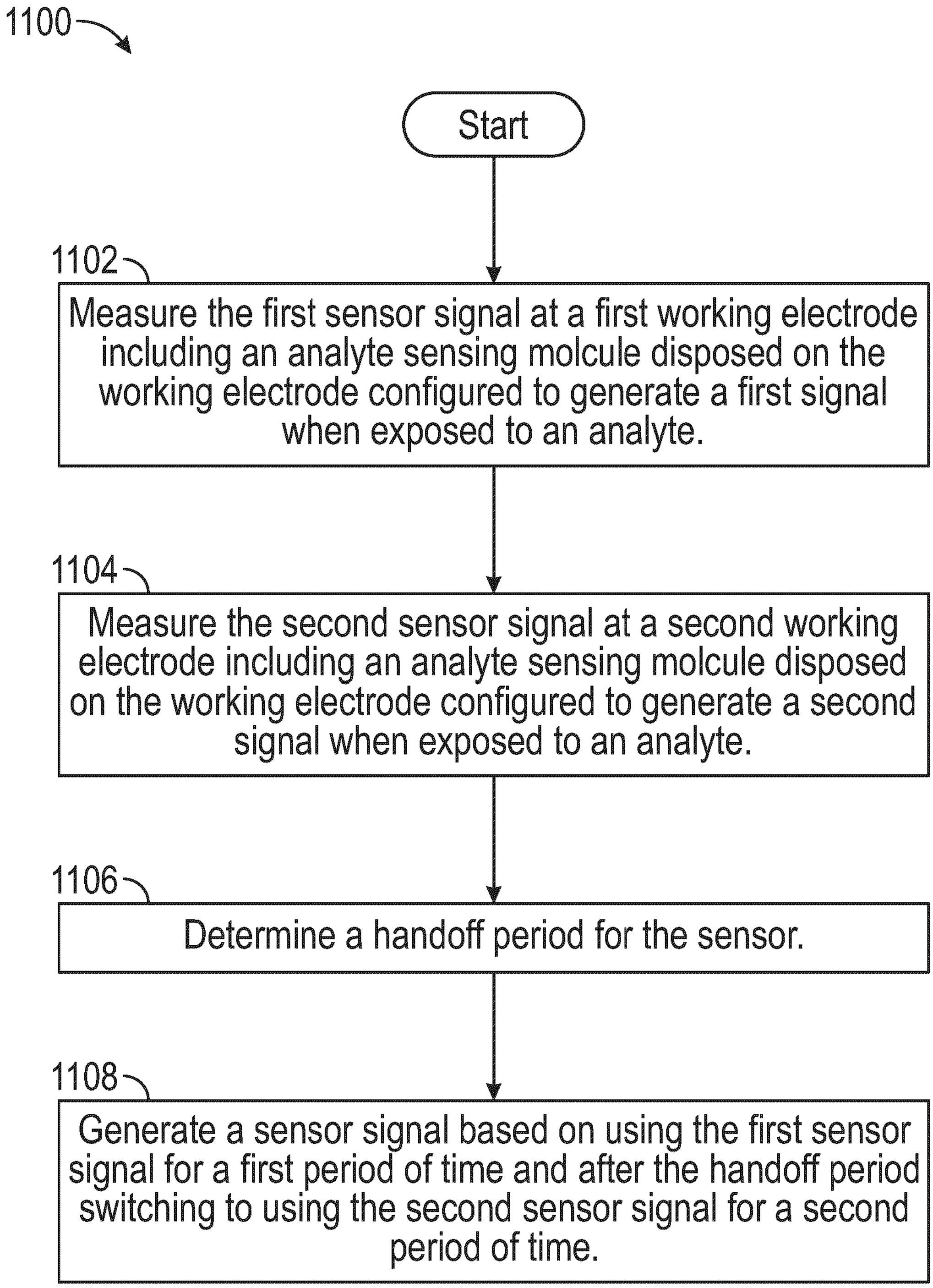
1100
Start
1102
Measure the first sensor signal at a first working electrode including an analyte sensing molcule disposed on the working electrode configured to generate a first signal when exposed to an analyte.
1104
Measure the second sensor signal at a second working electrode including an analyte sensing molcule disposed on the working electrode configured to generate a second signal when exposed to an analyte.
1106
Determine a handoff period for the sensor.
1108
Generate a sensor signal based on using the first sensor signal for a first period of time and after the handoff period switching to using the second sensor signal for a second period of time.

FIG. 10

Europäisches Patentamt
European Patent Office
Office européen des brevets

# EUROPEAN SEARCH REPORT

Application Number
EP 25 19 1014

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/097554 A1 (SHAH RAJIV [US] ET AL) 26 April 2012 (2012-04-26)<br>* paragraphs [0038] - [0039], [0041] - [0048], [0054], [0062] - [0065] *<br>* figures 2A-2c *<br>----- | 1-15 | INV.<br>A61B5/145<br>A61B5/1468<br><br>ADD.<br>A61B5/053 |
| X | US 2023/360799 A1 (NOGUEIRA KEITH [US] ET AL) 9 November 2023 (2023-11-09)<br>* paragraphs [0159], [0167], [0235] - [0239], [0565] - [0566] *<br>* figures 1, 2A *<br>----- | 1-15 | |

TECHNICAL FIELDS SEARCHED (IPC)
A61B

The present search report has been drawn up for all claims

1

| Place of search | Date of completion of the search | Examiner |
|---|---|---|
| The Hague | 28 August 2025 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 19 1014

This annex lists the patent family members relating to the patent documents cited in the above-mentioned European search report. The members are as contained in the European Patent Office EDP file on
The European Patent Office is in no way liable for these particulars which are merely given for the purpose of information.

28-08-2025

| Patent document cited in search report | Publication date | Patent family member(s) | Publication date |
|---|---|---|---|
| US 2012097554 A1 | 26-04-2012 | NONE | |
| US 2023360799 A1 | 09-11-2023 | CA 3008640 A1 | 06-07-2017 |
| | | CN 108697384 A | 23-10-2018 |
| | | EP 3397160 A1 | 07-11-2018 |
| | | US 2017185733 A1 | 29-06-2017 |
| | | US 2023360799 A1 | 09-11-2023 |
| | | WO 2017116505 A1 | 06-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63674023
- US 4562751 A
- US 4678408 A
- US 4685903 A
- US 4573994 A
- US 5391250 A
- US 5482473 A
- US 9642568 B